# EUROPEAN PATENT APPLICATION

(11) **EP 3 278 797 A1**
(43) Date of publication of application: **07.02.2018**
(21) Application number: 16773160.3
(22) Date of filing: 31.03.2016
(51) Int. Cl.: A61K 31/165, A61P 11/02, A61P 11/06, A61P 17/00, A61P 37/08

(54) **ANTIALLERGIC AGENT**

(30) Priority: 31.03.2015 JP 2015072034
(71) Applicant: Tohoku University, Sendai-shi, Miyagi 980-8577 (JP)
(72) Inventor: HIRASAWA, Noriyasu, Sendai-shi Miyagi 980-8577 (JP); WOO, Je-Tae, Kasugai-shi Aichi 487-8501 (JP); CHA, Byung-Yoon, Kasugai-shi Aichi 487-8501 (JP); YONEZAWA, Takayuki, Kasugai-shi Aichi 487-8501 (JP)
(74) Representative: Winter, Brandl, Fürniss, Hübner, Röss, Kaiser, Polte - Partnerschaft mbB
(86) International application number: PCT/JP2016/060764
(87) International publication number: WO 2016/159280

(57) **Abstract**

An object to be achieved by the present invention is to provide a novel antiallergic agent using, as an active ingredient, a compound that has heretofore not been known to have an antiallergic action. The present invention provides an antiallergic agent, including a compound represented by the formula (I) or a salt thereof: 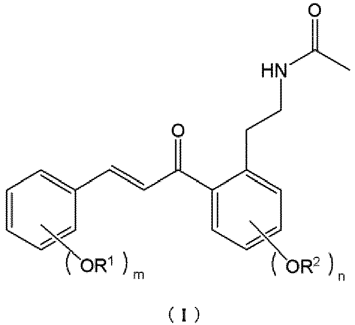 where R¹ and R², which are identical to or different from each other, each represent an alkyl group having 1 to 3 carbon atoms, m represents an integer of from 0 to 3, and n represents an integer of from 1 to 3.

## Description

### Technical Field

The present invention relates to an antiallergic agent targeted against an allergic disease, for example, atopic dermatitis.

### Background Art

### [Cross-reference to Related Application]

The present application claims priority from Japanese Patent Application No. 2015-072034, filed on March 31, 2015, the entire disclosure of which is incorporated herein by reference. Thymic stromal lymphopoietin (TSLP) is an IL-7-like cytokine isolated from a culture supernatant of thymic stromal cells. TSLP has attracted attention as a master switch for allergy development. It is known that local expression of TSLP in the lung, the skin, or the like induces an immune reaction of Th2 to develop an allergic disease, for example, asthma or atopic dermatitis. Therefore, TSLP serves as a novel potential drug target aimed at preventing the allergic disease and its exacerbation.

When production of TSLP can be suppressed, an antiallergic action is expected. Accordingly, research has heretofore been conducted into compounds having such action (Non Patent Literatures 1 and 2). However, any such compound needs to be administered at extremely high concentration in order to suppress the production of TSLP, and its effect has not been sufficient.

### Citation List

### Non Patent Literature

[NPL 1] Eur. J. Pharmacol. 671 (2011) 128-132
[NPL 2] Food Chemistry 133 (2012) 76-81

### Summary of Invention

### Technical Problem

An object to be achieved by the present invention is to provide a novel antiallergic agent using, as an active ingredient, a compound that has heretofore not been known to have an antiallergic action.

### Solution to Problem

Under such circumstances, the inventors of the present invention have made extensive investigations, and as a result, have found that, among compounds each having a chalcone skeleton represented by the following formula:

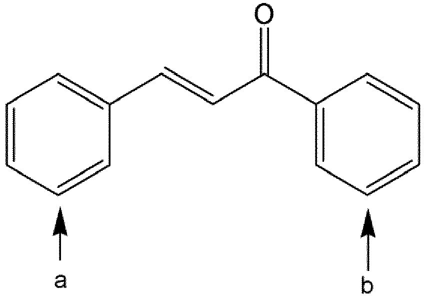

a compound that has a structure having an acetylaminoethyl group at an ortho position of a benzene ring b in the formula and further having an alkoxy group on the benzene ring b has a TSLP production inhibitory effect. The present invention is based on such novel finding.

Thus, the present invention provides the following items:
Item1. An antiallergic agent, including a compound represented by the following formula (I) or a salt thereof: 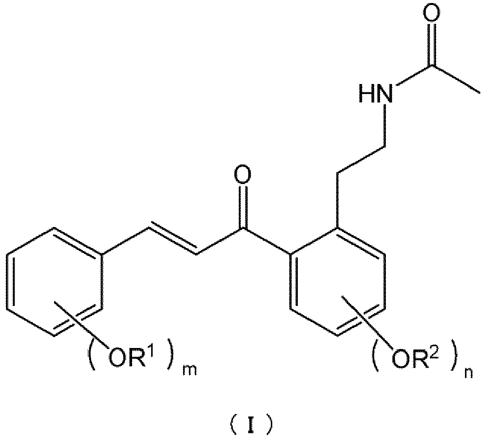 where R¹ and R², which are identical to or different from each other, each represent an alkyl group having 1 to 3 carbon atoms, m represents an integer of from 0 to 3, and n represents an integer of from 1 to 3.
Item 2. An antiallergic agent according to Item 1, in which m represents an integer of 1 or 2.
Item 3. An antiallergic agent according to Item 1 or 2, in which n represents 2.
Item 4. An antiallergic agent according to any one of Items 1 to 3, in which a target disease of the antiallergic agent includes at least one kind selected from the group consisting of atopic dermatitis, allergic bronchial asthma, and allergic rhinitis.
Item 5. A therapeutic method for an allergic disease, including a step of administering, to a mammal, a compound represented by the following formula (I) or a salt thereof: 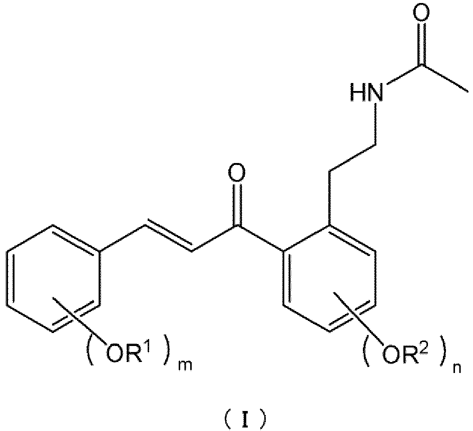 where R¹ and R², which are identical to or different from each other, each represent an alkyl group having 1 to 3 carbon atoms, m represents an integer of from 0 to 3, and n represents an integer of from 1 to 3.
Item 6. A use of a compound represented by the following formula (I) or a salt thereof, for production of an antiallergic agent: 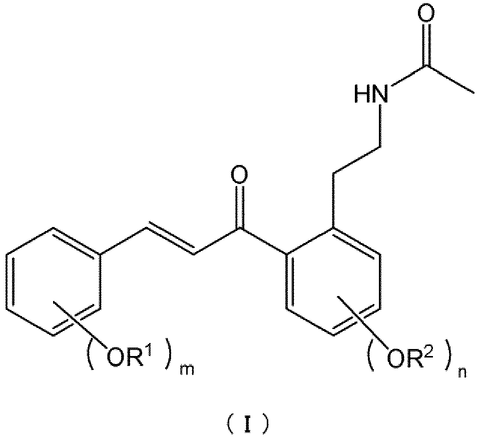 where R¹ and R², which are identical to or different from each other, each represent an alkyl group having 1 to 3 carbon atoms, m represents an integer of from 0 to 3, and n represents an integer of from 1 to 3.
Item 7. A compound represented by the following formula (I) or a salt thereof, for use in treatment of an allergic disease: 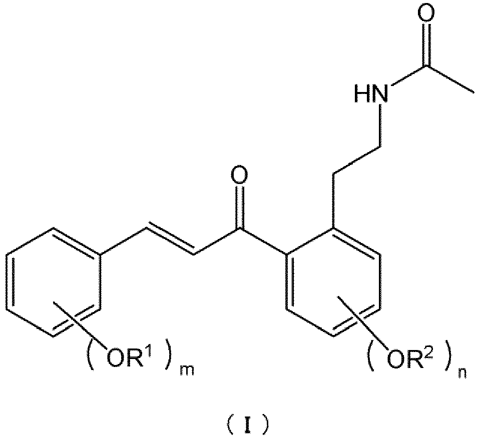 where R¹ and R², which are identical to or different from each other, each represent an alkyl group having 1 to 3 carbon atoms, m represents an integer of from 0 to 3, and n represents an integer of from 1 to 3.
Item 8. A method, a use, or a compound or a salt thereof according to any one of Items 5 to 7, in which m represents an integer of 1 or 2.
Item 9. A method, a use, or a compound or a salt thereof according to any one of Items 5 to 8, in which n represents 2.
Item 10. A method, a use, or a compound or a salt thereof according to any one of Items 5 to 9, in which a target disease of the compound or the salt includes at least one kind selected from the group consisting of atopic dermatitis, allergic bronchial asthma, and allergic rhinitis. Herein, the compound represented by the general formula (I) is sometimes referred to simply as compound (I).

### Advantageous Effects of Invention

The compound (I) or the salt thereof, which serves as the active ingredient of the present invention, has a TSLP production-suppressing effect. Thus, the present invention provides the novel antiallergic agent for treating allergic symptoms through the suppression of the production of TSLP. In addition, the compound (I) or the salt thereof, which serves as the active ingredient of the present invention, is predicted to have a novel action mechanism without suppressing the transcriptional activity of NFκB and AP-1, which have heretofore been known to contribute to the production of TSLP. Further, the compound (I) or the salt thereof, which serves as the active ingredient of the present invention, is effective also in suppressing the TSLP production at a lower concentration than a hitherto reported compound. In addition, cells that produce TSLP are mainly epithelial cells. Therefore, unlike an antiallergic agent targeted against a factor other than TSLP, the antiallergic agent of the present invention is expected to be effective as an external preparation and not to induce immunocompromisation unlike hitherto known anti-inflammatory and immunosuppressive drugs in terms of site of action. In addition, some steroidal anti-inflammatory drugs cause side effects, such as infectious diseases, due to excessive immunosuppressive actions. However, the compound (I) or the salt thereof, which serves as the active ingredient of the present invention, has high specificity for TSLP and a low anti-inflammatory action, and hence hardly causes side effects, such as infectious diseases, due to the expression of an excessive immunosuppressive action.

### Brief Description of Drawings

FIGS. 1 are graphs for showing TSLP mRNA expression levels obtained in Example 1.
FIG. 2 is a graph for showing test results of Example 2.
FIGS. 3 are graphs for showing test results of Example 3.
FIGS. 4 are graphs for showing test results of Example 4.
FIGS. 5 are graphs for showing the structures and test results of compounds used in Example 5.
FIGS. 6 are graphs for showing the structures and test results of the compounds used in Example 5.
FIG. 7 is a graph for showing the structure and test results of a compound used in Example 5.
FIGS. 8 are tables for showing inhibition ratios exhibited by 16D10 against various kinases measured in Example 6.
FIG. 9 is a graph for showing test results of Example 7.
FIG. 10 is an image for showing test results of Example 8.
FIGS. 11 are graphs for showing test results of Example 9.
FIG. 12 is a table for showing the structures and IC50's of compounds used in Example 10.
FIGS. 13 are graphs for showing test results of Example 11.

### Description of Embodiments

The present invention provides an antiallergic agent, including a compound represented by the following formula (I) or a salt thereof: 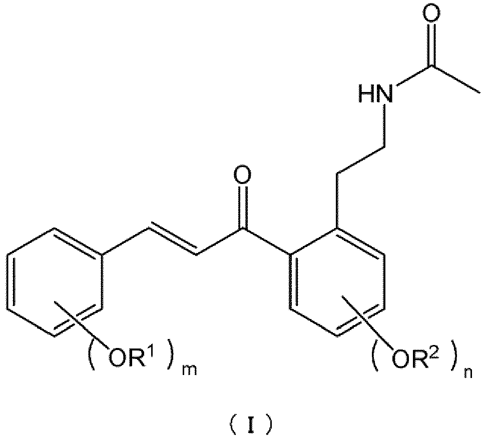 where R¹ and R², which are identical to or different from each other, each represent an alkyl group having 1 to 3 carbon atoms, m represents an integer of from 0 to 3, and n represents an integer of from 1 to 3.

In the general formula (I), examples of the alkyl group having 1 to 3 carbon atoms represented by each of R¹ and R² include a methyl group, an ethyl group, a n-propyl group, and an isopropyl group. Of the alkyl groups each having 1 to 3 carbon atoms, an alkyl group having 1 or 2 carbon atoms is preferred, and a methyl group is more preferred. In the present invention, R¹ and R² may be identical to or different from each other. In addition, when m represents 2 or more, the plurality of R¹'s may be identical to or different from each other. Similarly, when n represents 2 or more, the plurality of R²'s may be identical to or different from each other.

In the general formula (I), m represents an integer of from 0 to 3, preferably an integer of 1 or 2. When m represents 2 or more, it is preferred that the plurality of OR¹'s be not bonded to adjacent carbon atoms out of the constituent carbon atoms of the benzene rings, in other words, be bonded to carbon atoms that are separated by one or more carbon atoms out of the constituent carbon atoms of the benzene rings. Specifically, for example, when m represents 2, preferred to 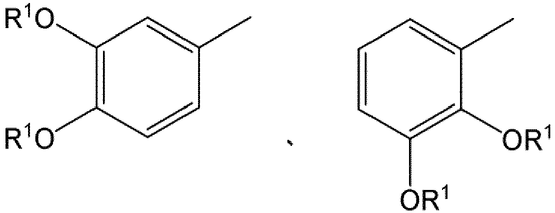 are 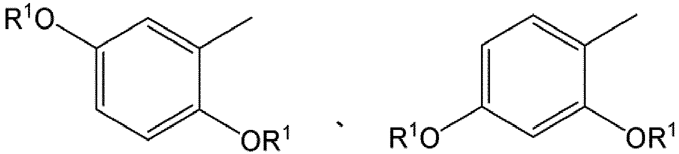 and the like.

In the general formula (I), n represents an integer of from 1 to 3, preferably 2. When n represents 2 or more, the plurality of OR²'s may be bonded to adjacent carbon atoms out of the constituent carbon atoms of the benzene rings, or may be bonded to a plurality of carbon atoms that are separated by one or more carbon atoms out of the constituent carbon atoms of the benzene rings.

The compound (I) serving as the active ingredient of the present invention may be known per se, or may be produced in accordance with a known method. For example, a compound 16D10 ((E)-N-[2-[2-[3-(2,5-dimethoxyphenyl)-1-oxo-2-propen-1-yl]-4,5 -dimethoxyphenyl]ethyl]acetamide) used in Examples to be described later is a known compound registered under CAS NO. 858744-44-2.

As an allergic disease serving as a target disease of the antiallergic agent of the present invention, there are given, for example, atopic dermatitis, allergic bronchial asthma, and allergic rhinitis. The antiallergic agent of the present invention is preferably used as a therapeutic drug for atopic dermatitis.

The salt of the compound serving as the active ingredient of the present invention encompasses an acid addition salt and a salt with a base. Specific examples of the acid addition salt include: inorganic acid salts, such as a hydrochloride, a hydrobromide, a hydroiodide, a sulfate, a perchlorate, and a phosphate; organic acid salts, such as an oxalate, a malonate, a succinate, a maleate, a fumarate, a lactate, a malate, a citrate, a tartrate, a benzoate, a trifluoroacetate, an acetate, a methanesulfonate, a p-toluenesulfonate, and a trifluoromethanesulfonate; and acidic amino acid salts, such as a glutamate and an aspartate. Specific examples of the salt with a base include: alkali metal or alkaline earth metal salts, such as a sodium salt, a potassium salt, and a calcium salt; salts with organic bases, such as a pyridine salt and a triethylamine salt; and salts with basic amino acids, such as lysine and arginine.

The compound (I) and the salt thereof, which serve as the active ingredients of the present invention, may each be present in the form of a hydrate or a solvate. Accordingly, the hydrate and the solvate are also encompassed in the compound serving as the active ingredient of the present invention. In addition, the compound (I) has a carbon-carbon double bond in the divalent group -CH=CH-(C=O)- linking two benzene rings, and hence may have geometrical isomers. Unless otherwise stated, those geometrical isomers are encompassed in the compound (I) serving as the active ingredient of the present invention (of those, an (E) isomer (trans form) is preferred).

As a solvent forming the solvate, there are given, for example: water; alcohols, such as ethanol and propanol; organic acids, such as acetic acid; esters, such as ethyl acetate; ethers, such as tetrahydrofuran and diethyl ether; ketones, such as acetone; and DMSO.

In the present invention, the compound (I) or the salt thereof, which serves as the active ingredient of the present invention, may be used, as it is, as an antiallergic agent, or may be used as a pharmaceutical composition in combination with any of various pharmaceutically acceptable carriers (for example, a tonicity agent, a chelating agent, a stabilizing agent, a pH adjuster, an antiseptic, an antioxidant, a solubilizing agent, and a thickening agent).

Examples of the tonicity agent include: saccharides, such as glucose, trehalose, lactose, fructose, mannitol, xylitol, and sorbitol; polyhydric alcohols, such as glycerin, polyethylene glycol, and propylene glycol; and inorganic salts, such as sodium chloride, potassium chloride, and calcium chloride.

Examples of the chelating agent include: edetates, such as disodium edetate, calcium disodium edetate, trisodium edetate, tetrasodium edetate, and calcium edetate; ethylene diamine tetraacetates; nitrilotriacetic acid or salts thereof; sodium hexametaphosphate; and citric acid.

An example of the stabilizing agent is sodium hydrogen sulfite.

Examples of the pH adjuster include: acids, such as hydrochloric acid, carbonic acid, acetic acid, and citric acid; and bases, for example: alkali metal hydroxides, such as sodium hydroxide and potassium hydroxide; alkali metal carbonates or hydrogen carbonates, such as sodium carbonate; alkali metal acetates, such as sodium acetate; alkali metal citrates, such as sodium citrate; and trometamol.

Examples of the antiseptic include: sorbic acid; potassium sorbate; parahydroxybenzoic acid esters, such as methyl parahydroxybenzoate, ethyl parahydroxybenzoate, propyl parahydroxybenzoate, and butyl parahydroxybenzoate; quaternary ammonium salts, such as chlorhexidine gluconate, benzalkonium chloride, benzethonium chloride, and cetylpyridinium chloride; alkylpolyaminoethylglycine; chlorobutanol; Polyquad; polyhexamethylene biguanide; and chlorhexidine.

Examples of the antioxidant include sodium hydrogen sulfite, dried sodium sulfite, sodium pyrosulfite, and mixed tocopherol concentrate.

Examples of the solubilizing agent include sodium benzoate, glycerin, D-sorbitol, glucose, propylene glycol, hydroxypropylmethylcellulose,polyvinylpyrrolidone,macrogol,and D-mannitol.

Examples of the thickening agent include polyethylene glycol, methylcellulose, ethylcellulose, carmellose sodium, xanthane gum, sodium chondroitin sulfate, hydroxyethylcellulose, hydroxypropylcellulose, hydroxypropylmethylcellulose, polyvinylpyrrolidone, and polyvinyl alcohol.

In addition, the pharmaceutical composition may further contain a compound known to have an antiallergic action, in addition to the compound (I) or the salt thereof.

In a pharmaceutical composition according to an embodiment of the present invention, the content of the compound (I) or the salt thereof in the composition is not particularly limited, and may be appropriately set within, for example, the following condition: 90 mass% or more, 70 mass% or more, 50 mass% or more, 30 mass% or more, 10 mass% or more, 5 mass% or more, or 1 mass% or more in terms of content of the compound (I).

A dosage form is not particularly limited, and examples thereof may include various dosage forms including: orally administered agents, such as a tablet, a pill, a capsule, a powder, a granule, and a syrup; and parenterally administered agents, such as injections (an intravenous injection,an intramuscular injection, a local injection, and the like), a gargle, a drop, external preparations (an ointment, a cream, a patch, and an inhalant), and a suppository. Of the above-mentioned dosage forms, preferred examples include external preparations (an ointment, a cream, a patch, and an inhalant). The compound (I) or the salt thereof, which serves as the active ingredient of the present invention, has a TSLP production-suppressing effect. The compound (I) or the salt thereof, which serves as the active ingredient of the present invention, is targeted against TSLP produced by epithelial cells instead of being targeted against lymphocytes, and hence can be expected to provide an antiallergic effect when used as an external preparation.

The content of the compound (I) of the present invention in a formulation differs depending on, for example, its administration route, and the age, body weight, and symptoms of a patient, and cannot be unconditionally specified. However, the content may be set to such an amount that the daily dose of the compound (I) is generally from about 10 mg to about 5,000 mg, more preferably from about 100 mg to about 1,000 mg. When the compound (I) is administered once a day, the above-mentioned amount may be contained in a single formulation, and when the compound (I) is administered 3 times a day, a third of the amount may be contained in a single formulation.

The antiallergic agent of the present invention is administered to patients including mammals. Examples of the mammals include a human, a monkey, a mouse, a rat, a rabbit, a cat, a dog, a pig, a bovine, a horse, and a sheep.

The antiallergic agent of the present invention alleviates allergic symptoms through the suppression of the production of TSLP. Therefore, the present invention also provides a TSLP production-suppressing agent including the compound (I) or the salt thereof. The active ingredient, dosage form, dose, and the like of the TSLP production-suppressing agent are similar to those of the antiallergic agent.

Now, the present invention is more specifically described by way of Examples below. However, the present invention is not limited thereto.

### Examples

### Example 1 16D10 suppresses production of TSLP even in human keratinocytes

HaCaT cells were suspended in 10% FBS-containing Dulbecco's MEM so as to have a cell count of 1.0×10⁵ cells/mL, and the suspension was seeded into each well of a 12-well plate (Falcon (trademark)) at 1,000 µl per well, followed by culture at 37°C under saturated humidity in the presence of 5% CO₂ until the cells achieved 80% to 90% confluence in each well. The culture solution was removed, and the cells were washed once with PBS that had been warmed to 37°C. After that, 1 ml of 10% FBS-DMEM containing 16D10 (10 and 100µM) and any of various stimulants [Poly(I:C) (50 µg/ml) (A), TNF-α (100 ng/ml) (B), and TNF-α (100 ng/ml)+IL-4 (10 ng/ml) (C)] was added, followed by further culture at 37°C under saturated humidity in the presence of 5% CO₂ for 4 hours. The final concentration of DMSO in each group was set to 0.1%.

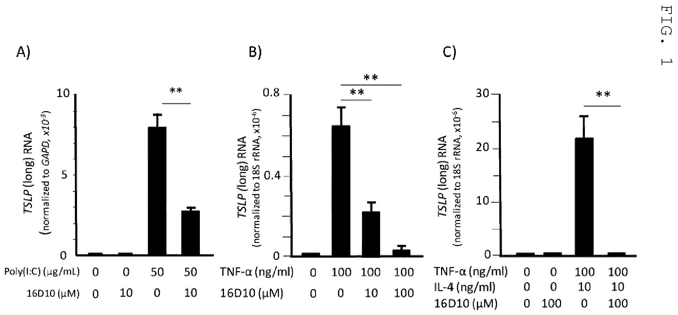

After the culture, the culture solution was removed, and the cells were washed twice with PBS. After that, total RNA was extracted with RNAiso Plus. A reverse transcription reaction was performed with PrimeScript (trademark) RT Master Mix (Perfect Real Time) in accordance with an accompanying protocol. A real-time PCR reaction was performed with SYBR (trademark) Premix EX Taq™ II (Tli RNaseH Plus) in accordance with an accompanying protocol. The following primers were used: Human TSLP (long) Forward: 5'-GAT TAC ATA TAT GAG TGG GAC-3', Reverse: 5'-TTC ATT GCC TGA GTA GCA T-3', GAPDH Forward: 5'-TGT GTC CGT CGT GGA TCT GA-3', Reverse: 5'-TTG CTG TTG AAG TCG CAG GAG-3', 18S rRNA forward: 5'-TTGACGGAAGGGCACCACCAG-3', Reverse: 5'-GCACCACCACCCACGGAATCG-3'. The threshold cycle (Ct) value of each sample was calculated by a 2nd derivative maximum method, and a relative calibration curve was created to confirm that amplification efficiency under PCR reaction conditions for each gene was close to 100%. After that, the Ct value was converted to an RNA relative amount by a Ct method. The results are shown in FIGS. 1. 16D10 suppressed the expression of TSLP mRNAby stimulation with each of poly(I:C), TNF-α, and TNF-α+IL-4 in the human keratinocyte cell line.

### Example 2 16D10 suppresses TSLP production by TNF-α stimulation in mouse keratinocyte PAM212 cells

The mouse keratinocyte PAM212 cell line was suspended in 10% FBS-containing Dulbecco' s MEM so as to have a cell count of 1.0×10⁵ cells/mL, and the suspension was seeded into each well of a 12-well plate (Falcon (trademark)) at 1,000 µl per well, followed by culture at 37°C under saturated humidity in the presence of 5% CO₂ until the cells achieved 80% to 90% confluence in each well. The culture solution was removed, and the cells were washed once with PBS that had been warmed to 37°C. After that, 1 ml of 10% FBS-DMEM containing 16D10 (1 µM, 3 µM, and 10 µM) with or without TNF-α (30 ng/ml) was added, followed by further culture at 37°C under saturated humidity in the presence of 5% CO₂ for 48 hours. The final concentration of DMSO in each group was set to 0.1%.

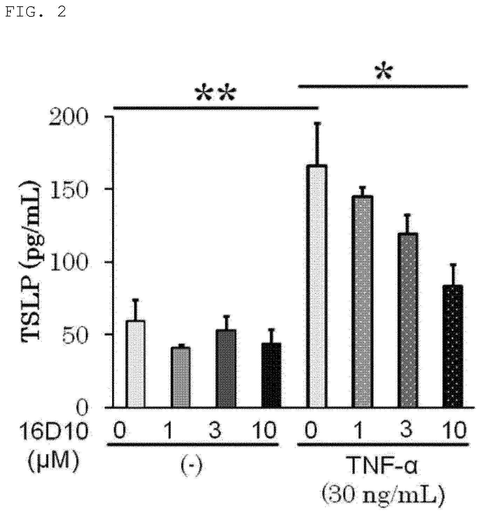

After the culture, TSLP in the culture solution was measured by EILSA. The results are shown in FIG. 2.

16D10 suppressed TSLP production by TNF-α in a concentration-dependent manner.

### Example 3 16D10 suppresses TSLP production also in vivo in mouse

500 µl of air was subcutaneously injected into the back of a Balb/c mouse (6-week-old, male, SLC) to form an air pouch (day 1). Further, 500 µl each of air was injected into the air pouch on day 2, day 5, and day 7. 24 hours after that, 300 µl of a solution prepared by dissolving LPS at 10 ng/ml in 2% (w/v) CMC-Na physiological saline that had been subjected to high-pressure steam sterilization and further dissolving penicillin G potassium and streptomycin sulfate at 0.1 mg/ml each therein was injected into the air pouch to induce inflammation.

16D10 and dexamethasone (0.5 µg/ml) were dissolved in DMSO, and then mixed in LPS-containing CMC-Na physiological saline so that the concentration of DMSO was 0.5% (v/v). The resultant was stirred well, and then administered into the air pouch.

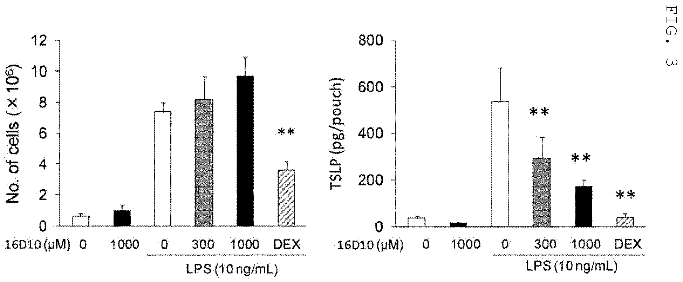

8 hours after the administration of the solution into the air pouch, the mouse was killed through blood removal by cutting the carotid artery under anesthesia. 300 µl of physiological saline was injected into the air pouch so as to be mixed with air pouch internal fluid, and then the air pouch internal fluid was collected and its weight was measured. An infiltrating leukocyte count was measured with a hemocytometer after the collected air pouch internal fluid had been diluted with physiological saline as appropriate. The remaining air pouch internal fluid was centrifuged at 4°C for 10 minutes at 1,490×g. The supernatant was collected, and its TSLP concentration was measured with Mouse TSLP DuoSet (trademark) ELISA (R&D Systems) in accordance with an accompanying protocol. The results are shown in FIGS. 3 (left graph of FIGS. 3: infiltrating leukocyte count, right graph of FIGS. 3: TSLP concentration). As apparent from the right graph of FIGS. 3, 16D10 significantly suppressed TSLP production. Meanwhile, as apparent from the left graph of FIGS. 3, 16D10 did not reduce infiltrating leucocytes, and did not exhibit an inflammation-suppressing action.

### Example 4 16D10 suppresses production of IgE in mouse

500 µl of air was subcutaneously injected into the back of an ICR mouse (6-week-old, male, SLC) to form an air pouch (day 1). Further, 500 µl each of air was injected into the air pouch on day 2, day5, and day 7 . 24 hours after that, 300 µl of a solution prepared by dissolving ovalbumin (OVA) at 100 µg/0.3 ml in 2% (w/v) CMC-Na physiological saline that had been subjected to high-pressure steam sterilization and further dissolving penicillin G potassium and streptomycin sulfate at 0.1 mg/ml each therein was injected into the air pouch to induce inflammation. 16D10 was dissolved in DMSO, and then mixed in OVA-containing CMC-Na physiological saline so that the concentration of DMSO was 0.5% (v/v). The resultant was stirred well, and then administered into the air pouch.

8 hours after the administration of the solution into the air pouch, the mouse was killed through blood removal by cutting the carotid artery under anesthesia. 300 µl of physiological saline was injected into the air pouch so as to be mixed with air pouch internal fluid, and then the air pouch internal fluid was collected and its weight was measured. Its TSLP concentration was measured with Mouse TSLP DuoSet (trademark) ELISA (R&D Systems) in accordance with an accompanying protocol. 14 days after the administration of the solution into the air pouch, blood was collected, and the amount of OVA-specific IgE in serum was measured with an ELISA kit (Anti-ovalbumin IgE (mouse) EIA kit (Cayman 500840)).

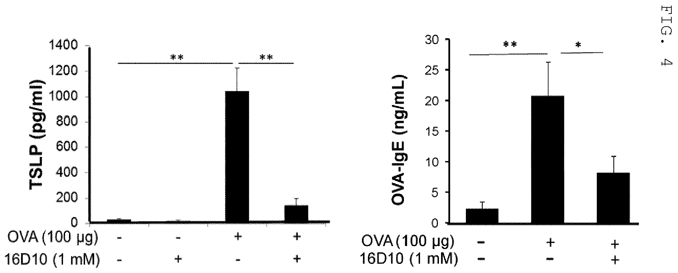

The results are shown in FIGS. 4. As apparent from the right graph of FIGS. 4, 16D10 significantly suppressed TSLP production by OVA. In addition, as apparent from the left graph of FIGS. 4, 16D10 suppressed the production of OVA-specific IgE by OVA administration.

### Example 5 Structure-activity Correlation

KCMH-1 cells (J. Immunol. Methods 402: 9-14 (2013)) were suspended in 10% FBS-containing MEM-α at 1.0×10⁵ cells/mL. 500 µl of the suspension was seeded into each well of a 24-well plate (Falcon (trademark)), followed by culture at 37°C under saturated humidity in the presence of 5% CO₂ until the cells achieved 80% to 90% confluence in each well.

The cells were washed once with PBS that had been warmed to 37°C, and then cultured in 10% FBS-containing MEMα containing a drug at various concentrations for 24 hours. The culture supernatant was collected, and a TSLP amount was measured with Mouse TSLP DuoSet (trademark) ELISA (R&D Systems).

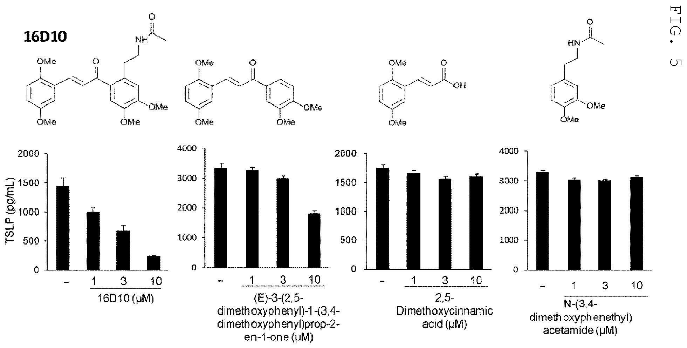

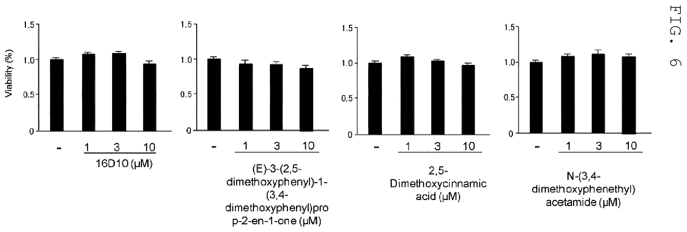

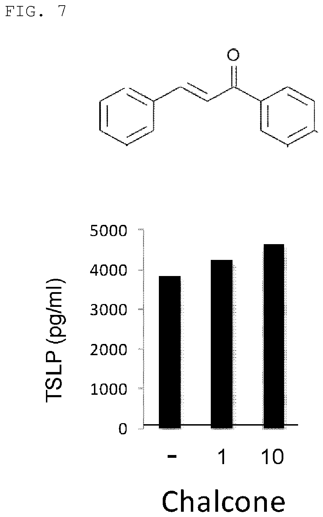

The culture supernatant of the cells was collected. After that, 0.5% MTT was diluted 10-fold with medium and added to each well, followed by culture at 37°C under saturated humidity in the presence of 5% CO₂ for from 1 hour to 4 hours. After that, the culture supernatant was removed, and an equal amount of DMSO was added to dissolve formazan in the cells. The absorbance of the solution at 570 nm was measured with a microplate reader (iMarkTN, Bio-Rad), and the viability of the cells was calculated. The results are shown in FIGS. 5, FIGS. 6, and FIG. 7. As shown in FIGS. 5 and FIG. 7, 16D10 exhibited an extremely high TSLP production-suppressing ability as compared to (E)-3-(2,5-dimethoxyphenyl)-1-(3,4-dimethoxyphenyl)prop-2-en-1 -one, 2,5-dimethoxycinnamic acid, N-(3,4-dimethoxyphenethyl)acetamide, and chalcone. Meanwhile, as shown in FIGS. 6, the compounds did not significantly differ in viability.

### Example 6 16D10 does not suppress various kinases

Carna Biosciences, Inc. was entrusted to measure the effect of the test compound 16D10 at 10 µM on the activity of each kinase by Off-chip Mobility Shift Assay (MSA) described on the website of Carna Biosciences, Inc. (http://www.carnabio.com/output/pdf/ProfilingProfilingBook_ja. pdf). Specifically, the measurement was performed by the following method.

### Test Compound

The test compound is dissolved in and diluted with DMSO to a 100-fold concentration. Then, the solution is diluted 25-fold with assay buffer to prepare a test compound solution. A control is similarly prepared.

### Assay Reagent and Procedure

### Off-chip Mobility Shift Assay (MSA)

(1) 5 mL of a 4-fold compound solution, 5 mL of a 4-fold Substrate/ATP/Metal solution, and 10 mL of a 2-fold kinase solution are prepared with assay buffer (20 mM HEPES, 0.01% Triton X-100, 2 mM DTT, pH 7.5), and are mixed and incubated in a polypropylene 384-well plate at room temperature for from 1 hour or 5 hours* (*the reaction time is set depending on the kinase);
(2) 70 mL of termination buffer (QuickScout Screening Assist MSA; Carna Biosciences) is added to each well;
(3) the reaction mixture is applied to a LabChipTM system (PerkinElmer), and the peaks of a product and a substrate peptide are separated and quantified; and
(4) a kinase reaction is evaluated on the basis of a product ratio (P/(P+S)) calculated from the peak heights of the product (P) and the substrate (S) peptide.

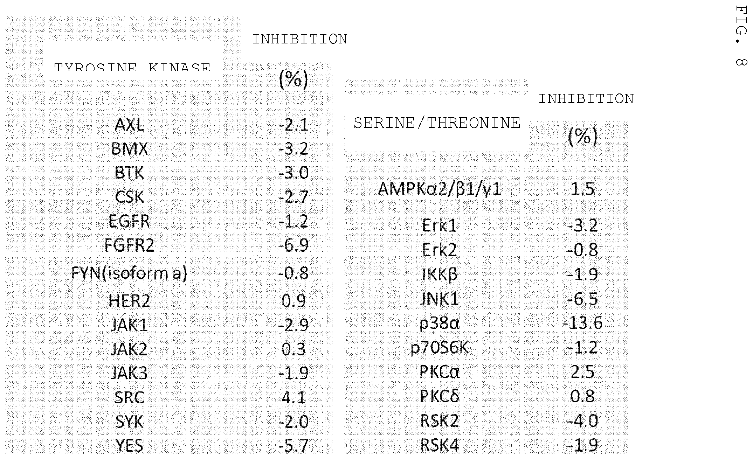

The reaction conditions are as shown in Table 1 and Table 2. The results are shown in FIGS.8. 16D10 did not suppress the activity of each kinase analyzed.

**Table 1**

| Kinase | Platform | Substrate | | ATP (µ M) | | Metal | | Positive control |
|---|---|---|---|---|---|---|---|---|
| | | Name | (nM) | Km | Assay | Name | (mM) | |
| AXL | MSA | CSKtide | 1000 | 32 | 50 | Mg | 5 | Staurosporine |
| BMX | MSA | Sretide | 1000 | 75 | 75 | Mg | 5 | Staurosporine |
| BTK | MSA | Sretide | 1000 | 72 | 75 | Mg | 5 | Staurosporine |
| CSK₁₎ | MSA | Sretide | 1000 | 4.8 | 5 | Mg+Mn | 5+1 | Staurosporine |
| EGFR | MSA | Sretide | 1000 | 2.7 | 5 | Mg+Mn | 5+1 | Staurosporine |
| FYN[isoform a] | MSA | Sretide | 1000 | 36 | 50 | Mg | 5 | Staurosporine |
| HER2 | MSA | Sretide | 1000 | 9.4 | 10 | Mn | 5 | Staurosporine |
| JAK1₁₎₂₎ | MSA | JAK1 substrate peptide | 1000 | 68 | 75 | Mg | 5 | Staurosporine |
| JAK2 | MSA | Sretide | 1000 | 13 | 10 | Mg | 5 | Staurosporine |
| JAK3 | MSA | Sretide | 1000 | 3.5 | 5 | Mg | 5 | Staurosporine |
| SRC | MSA | Sretide | 1000 | 31 | 50 | Mg | 5 | Staurosporine |
| SYK | MSA | Blk/Lyntide | 1000 | 26 | 25 | Mg | 5 | Staurosporine |
| YES(YES1) | MSA | Sretide | 1000 | 13 | 10 | Mg | 5 | Staurosporine |
| AMPKα2/β1/Y1 | MSA | SAMS peptide | 1000 | 100 | 100 | Mg | 5 | Staurosporine |
| Erk1 | MSA | Modified Erktide | 1000 | 34 | 50 | Mg | 5 | 5-Iodotubercidin |
| Erk2 | MSA | Modified Erktide | 1000 | 33 | 50 | Mg | 5 | 5-Iodotubercidin |
| IKKβ | MSA | Modified IκBα -derived peptide | 1000 | 16 | 25 | Mg | 5 | Staurosporine |
| JNK1 | MSA | Modified Erktide | 1000 | 29 | 100 | Mg | 5 | JNK Inhibitor II |
| p38α | MSA | Modified Erklide | 1000 | 150 | 150 | Mg | 5 | SB202190 |
| p70S6K | MSA | S6k2 | 1000 | 14 | 10 | Mg | 5 | Staurosporine |

**Table 2**

| | | | | | | | | |
|---|---|---|---|---|---|---|---|---|
| | | peptide | | | | | | |
| PKCα 3) | MSA | PKC peptide | 1000 | 36 | 50 | Mg+Ca | 5+0.05 | Staurosporine |
| PKCδ 3) | MSA | PKC peptide | 1000 | 26 | 25 | Mg | 5 | Staurosporine |
| RSK2 | MSA | S6K peptide (N-FL) | 1000 | 14 | 10 | Mg | 5 | Staurosporine |
| RSK4 | MSA | S6K peptide (N-FL) | 1000 | 20 | 25 | Mg | 5 | Staurosporine |

| | | | | | | | | |
|---|---|---|---|---|---|---|---|---|
| 1) Reaction time is 5 hours. 2) Sodium orthovanadate is added at the final concentration of 25 µM. 3) Phosphatidylserine and Diaryl Glycerol are added at the final concentration of 50 µg/mL and 5 µg/mL, respectively. | | | | | | | | |

### Example 7 16D10 does not suppress NFκB in human keratinocytes

The transfection of Path Detect pNF-κB-Luc Plasmid (Stratagane) was performed with X-tremeGENE HP DNA Transfection Reagent in accordance with an accompanying protocol. Human keratinocyte HaCaT cells were seeded at 0.65×10⁵ cells/ml into a 12-wellplate (Falcon (trademark)), and cultured for 24 hours. After 2 days, first, to 50 µl of Opti-MEM at from 15°C to 25°C, a 500 ng/µl reporter plasmid solution and a 100 ng/µl pGL4.74 [hRluc/TK] Vector solution were added at 1 µl each. Next, 1.5 µl of X-tremeGENE HP DNA Transfection Reagent was added, and the contents were lightly mixed by inversion and then left to stand still at room temperature for 15 minutes to form a transfection complex. Further, the culture supernatant was removed from the cells that had been cultured for 24 hours, and the cells were washed with PBS. After that, 950 µl of 10% FBS-containing MEMα was added to each well. 50 µl of the previously prepared transfection complex solution was added dropwise, and the plate was gently shaken, followed by culture at 37°C under saturated humidity in the presence of 5% CO₂ for 24 hours.

After that, 1 ml of 10% FBS-containing MEM containing TNF-α (100 ng/ml) and a drug (16D10, 10 µM or 100 µM, or [5-(p-Fluorophennyl)-2-ureido]thiophene-3-carboxamide (TPCA-1); IKK inhibitor, 10 µM) was added, followed by further culture for 2 hours. After that, the culture supernatant of the cells was removed, and the cells were washed twice with PBS. After that, 100 µl of 1×Passive lysis Buffer was added to detach adhering cells, followed by pipetting. After that, the whole amount of the cell lysate was collected as a sample in a 1.5 ml tube. The cell lysate was stored at -80°C, and freeze-thawed once before being used for measurement.

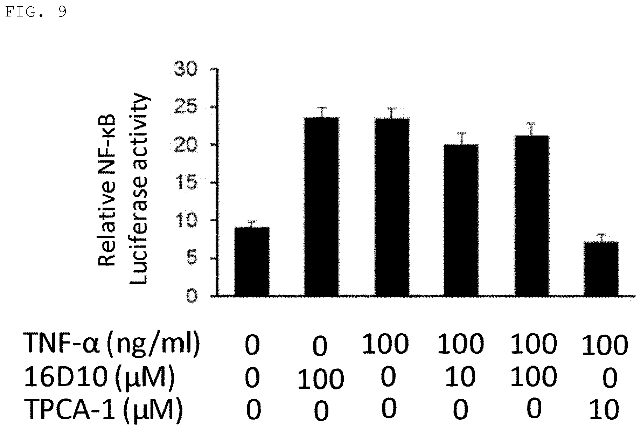

The measurement of luciferase activity was performed with Dual-Luciferase (trademark) Reporter Assay System in accordance with an accompanying protocol. Luciferase assay reagent II was dispensed at 50 µl each into tubes for measurement, and 10 µl of the cell lysate sample obtained by the above-mentioned method was added, followed by pipetting. Immediately after that, a Firefly luciferase luminescence integrated value for 10 seconds was calculated with AB-2200-R Luminescencer PSN (ATTO Corporation). After that, 50 µl of Stop&Glo reagent was added and the contents were lightly mixed, and then a Renilla luciferase luminescence integrated value for 10 seconds was measured. The results are shown in FIG. 9. The IKK inhibitor TPCA-1, capable of suppressing the activation of NF-κB, suppressed the transcriptional activity of NF-κB, but 16D10 did not suppress the activity.

### Example 8 16D10 does not suppress NF-κB-activating signal and tyrosine phosphorylation of STAT6 in human keratinocytes

HaCaT cells were seeded at 0.25×10⁵ cells/ml into each well of a 12 well plate, and cultured at 37°C under saturated humidity in the presence of 5% CO₂ for 2 days. The culture solution was removed, and the cells were washed once with PBS. After that, 1 ml of 10% FBS-DMEM containing 16D10 (100 µM) and TNF-α (100 ng/ml)+IL-4 (10 ng/ml) was added, followed by further culture at 37°C under saturated humidity in the presence of 5% CO₂ for 10 minutes. The cells were washed twice with PBS. After that, the cells were lysed with 100 µl of lysis buffer, and western blot was performed with Phospho-IKKα/β (Cell signaling, #2697), IKKα/β (SantaCruz, sc-7607), IκBα: (Cell signaling, #9242), Phospho-Stat6 (Cell signaling, #9361), and α-tubulin (SantaCruz, sc-5286) antibodies. Ahorsradishperoxidase label was used as a secondary antibody. For the detection of a protein of interest, chemiluminescence to be generated through the use of Western Lightning (trademark) Plus-ECL as a substrate was detected with Light Capture AE-6960 (ATTO Corporation).

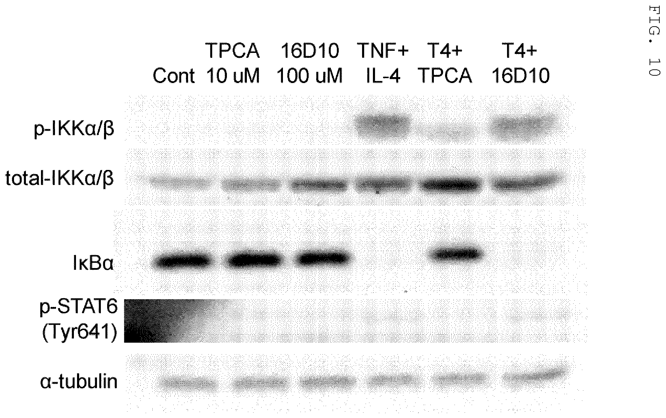

The results are shown in FIG. 10. The TNF-α+IL-4 stimulation-induced increase in phosphorylation of IKK and reduction in inhibitoryprotein IκB of NF-κB were suppressed by the IKK inhibitor TPCA, but were not suppressed by 16D10. In addition, 16D10 did not suppress the increase in phosphorylation of STAT6 by stimulation, either.

### Example 9 16D10 suppresses TSLP production without suppressing NFκB also in mouse keratinocytes

The transfection of Path Detect pNF-κB-Luc Plasmid (Stratagane) was performed with X-tremeGENE HP DNA Transfection Reagent in accordance with an accompanying protocol. Mouse keratinocyte KCMH-1 cells were seeded at 1×10⁵ cells/ml into a 12-well plate (Falcon (trademark)), and cultured for 24 hours. The following day, first, to 50 µl of Opti-MEM at from 15°C to 25°C, a 500 ng/µl reporter plasmid solution and a 100 ng/µl pGL4.74 [hRluc/TK] Vector solution were added at 1 µl each. Next, 1.5 µl of X-tremeGENE HP DNA Transfection Reagent was added, and the contents were lightly mixed by inversion and then left to stand still at room temperature for 15 minutes to form a transfection complex. Further, the culture supernatant was removed from the cells that had been cultured for 24 hours, and the cells were washed with PBS. After that, 950 µl of 10% FBS-containing MEMα was added to each well. 50 µl of the previously prepared transfection complex solution was added dropwise, and the plate was gently shaken, followed by culture at 37°C under saturated humidity in the presence of 5% CO₂ for 24 hours. The culture supernatant of the cells was removed, and 1 ml of 10% FBS-containing MEM containing a drug (16D10, 10 µM, [5-(p-Fluorophennyl)-2-ureido]thiophene-3-carboxamide (TPCA-1); IKK inhibitor) was added, followed by further culture for 24 hours. After that, the culture supernatant of the cells was removed, and the cells were washed twice with PBS. After that, 100 µl of 1×Passive lysis Buffer was added to detach adhering cells, followed by pipetting. After that, the whole amount of the cell lysate was collected as a sample in a 1.5 ml tube. The cell lysate was stored at -80°C, and freeze-thawed once before being used for measurement.

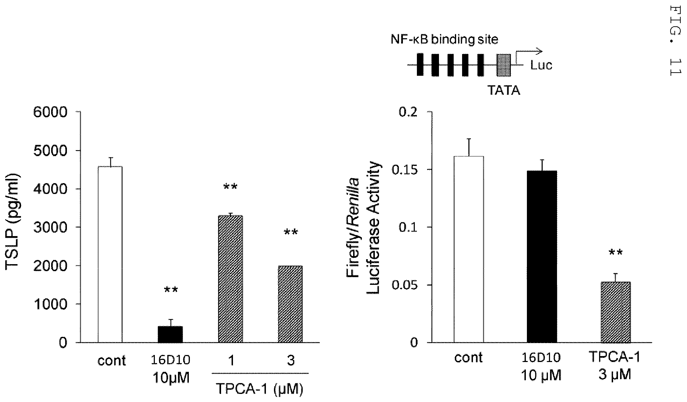

The measurement of luciferase activity was performed with Dual-Luciferase (trademark) Reporter Assay System in accordance with an accompanying protocol. Luciferase assay reagent II was dispensed at 50 µl each into tubes for measurement, and 10 µl of the cell lysate sample obtained by the above-mentioned method was added, followed by pipetting. Immediately after that, a Firefly luciferase luminescence integrated value for 10 seconds was calculated with AB-2200-R Luminescencer PSN (ATTO Corporation). After that, 50 µl of Stop&Glo reagent was added and the contents were lightly mixed, and then a Renilla luciferase luminescence integrated value for 10 seconds was measured. The results are shown in FIGS. 11. Also in the mouse keratinocyte cell line, 16D10 suppressed TSLP production without suppressing the transcriptional activity of NF-κB.

### Example 10 Investigation on Position of Methoxy Group

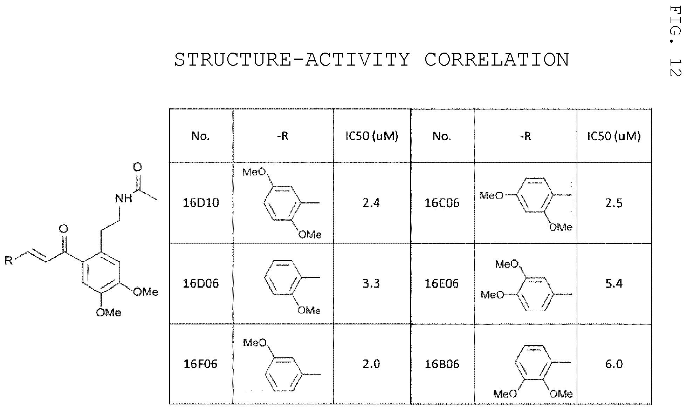

A TSLP amount was measured in the same manner as in Example 3 using compounds having structures shown in FIGS. 8, that is, 16D06 (N-(2-{2-[(2E)-3-(2-methoxyphenyl)prop-2-enoyl]-4,5-dimethoxyp henyl}ethyl)acetamide), 16F06 (N-(2-{2-[(2E)-3-(3-methoxyphenyl)prop-2-enoyl]-4,5-dimethoxyp henyl}ethyl)acetamide), 16C06 (N-(2-{2-[(2E)-3-(2,4-dimethoxyphenyl)prop-2-enoyl]-4,5-dimeth oxyphenyl}ethyl)acetamide), 16E06 (N-(2-{2-[(2E)-3-(3,4-dimethoxyphenyl)prop-2-enoyl]-4,5-dimeth oxyphenyl}ethyl)acetamide), and 16B06 (N-(2-{2-[(2E)-3-(2,3-dimethoxyphenyl)prop-2-enoyl]-4,5-dimeth oxyphenyl}ethyl)acetamide). Then, a compound concentration (IC50) at which the TSLP amount became 1/2 was calculated. The results are shown in FIG. 12 together with that of 16D10. As shown in FIG. 12, 16D10, 16D06, 16F06, 16C06, 16E06, and 16B06 each exhibited a high TSLP production-suppressing effect. In addition, of those, in particular, 16D10, 16D06, 16F06, and 16C06 exhibited high TSLP production-suppressing effects.

### Example 11

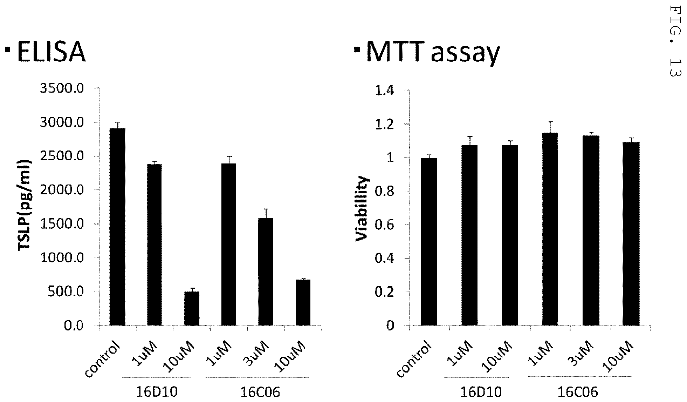

The compound 16C0 was subjected to the measurement of a TSLP amount and the measurement of viability by the above-mentioned MTT assay in the same manner as in Example 3. The results are shown in FIGS. 13.

### Industrial Applicability

The number of patients with allergic diseases has been increasing year by year, and hence there is a strong need for a therapeutic method therefor. The present invention provides the novel antiallergic agent using, as an active ingredient, a compound that has heretofore not been known to have an antiallergic action. In addition, the antiallergic agent of the present invention is predicted to have an action mechanism different from that of a known antiallergic agent. Therefore, the present invention is extremely useful in providing a novel antiallergic agent.

### Sequence Listing

PCT_anti-allergenic agent_20160331_102606_10.txt

## Claims

1. An antiallergic agent, comprising a compound represented by the following formula (I) or a salt thereof: 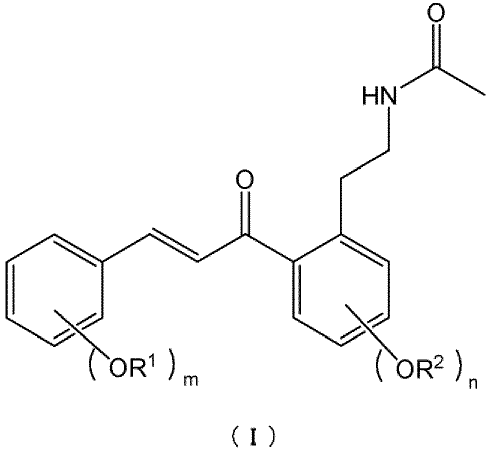 where R¹ and R², which are identical to or different from each other, each represent an alkyl group having 1 to 3 carbon atoms, m represents an integer of from 0 to 3, and n represents an integer of from 1 to 3.

2. An antiallergic agent according to claim 1, wherein m represents an integer of 1 or 2.

3. An antiallergic agent according to claim 1 or 2, wherein n represents 2.

4. An antiallergic agent according to any one of claims 1 to 3, wherein a target disease of the antiallergic agent comprises at least one kind selected from the group consisting of atopic dermatitis, allergic bronchial asthma, and allergic rhinitis.

5. A therapeutic method for an allergic disease, comprising a step of administering, to a mammal, a compound represented by the following formula (I) or a salt thereof: 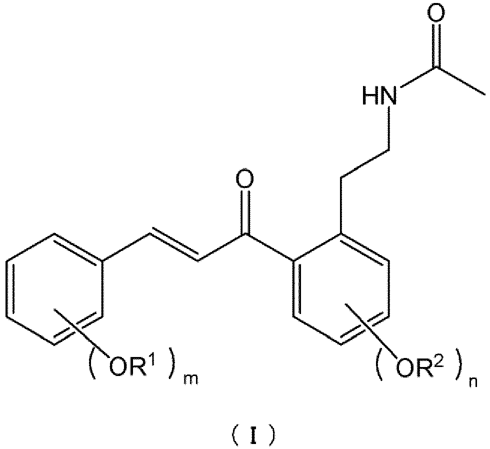 where R¹ and R², which are identical to or different from each other, each represent an alkyl group having 1 to 3 carbon atoms, m represents an integer of from 0 to 3, and n represents an integer of from 1 to 3.
